# EUROPEAN PATENT APPLICATION

(11) **EP 4 520 257 A1**
(43) Date of publication of application: **12.03.2025**
(21) Application number: 24198408.7
(22) Date of filing: 04.09.2024
(51) Int. Cl.: A61B 5/00, A61B 5/053

(54) **SENSORIZED SURGICAL DEVICE AND METHOD OF OPERATION OF SUCH A DEVICE**

(30) Priority: 11.09.2023 IT 202300018600
(71) Applicant: Università degli Studi di Roma "La Sapienza", 00185 Roma (IT)
(72) Inventor: D'ANDREA, Vito, 00187 Rome (IT); TRIFILETTI, Alessandro, 00187 Roma (IT); BELLINI, Maria Irene, 00187 Roma (IT); TOMMASINO, Pasquale, 00187 Roma (IT)
(74) Representative: Tiburzi, Andrea

(57) **Abstract**

The present invention relates to a sensorized device (1) for detecting conductive properties of an anatomical tissue, comprising a first jaw (11), a second jaw (12), mechanically connected to said first jaw (11) and articulated with respect to it, so that said tissue can be caught between said two jaws (11, 12), at least two application electrodes (4, 5) for applying an alternating current to said anatomical tissue, wherein said application electrodes (4, 5) are arranged on said jaws (11, 12), at least two measurement electrodes (6, 7), for detecting the impedance of said anatomical tissue, arranged on one of said jaws (11, 12), a light source (2) for emitting one or more light radiations on the anatomical tissue, and an optical receiver (3), configured to receive said light radiation to receive an optical spectrum of said anatomical tissue and generate a signal containing information on the optical spectrum, processing and control means (10, U), connected to said application electrodes (4, 5) and to said measurement electrodes (6, 7), and signaling means (13), connected to said processing and control means (10, U).

The present invention also relates to a method for detecting conductive properties of a tissue.

## Description

The present invention relates to a sensorized surgical device and method of operation of such a device.

### Field of the invention

More specifically, the invention relates to a device for surgical use, and its operating method, such as a forceps or the like, equipped with integrated sensors for the identification of cancerous tissue operating in real time, but which can be used for any medical circumstance in which there is a need for real-time detection of the condition of a tissue.

In the following, the description will be directed to a surgical forceps, but it is clear that it should not be considered limited to this specific use.

### Prior art

As is well known at present, the intraoperative cryostat examination, termed "extemporaneous" examination, is an anatomic-pathologic diagnosis performed during surgery on a fragment of the suspected neoplastic lesion, according to the result of which, the surgery itself can be conducted conservatively or demolition.

The application of said intraoperative examination is to clarify the diagnosis of the disease, in case this has not been previously made, or in case new diagnostic questions emerge in the operating room, or, finally, to assess the residual disease in the margins of the excised surgical piece, thus guiding the subsequent therapeutic strategy.

From a technical standpoint, the extemporaneous examination indicated above involves a biopsy of the surgical piece, with sampling and diagnosis at the same time as the surgery itself, but with often not easily reducible and predictable times, which inevitably burden the total operative time.

In fact, the extemporaneous examination involves numerous complex procedures that need to be carried out in a predetermined sequence: biopsy collection, freezing of the same inside the cryostat and cutting by a laboratory technician, preparation of the section for its adhesion on the object slide, staining and reading by the anatomo-pathologist for diagnosis.

It seems clear that the combination of these procedures, in addition to a considerable logistical effort, also requires highly qualified personnel and associated costs resulting from the use of dedicated equipment and space.

Specifically, the search for analysis methods that can enable real-time identification of cancerous tissue is a very active area of research in recent decades, and has led to the publication of several scientific papers and patent application filings. The methodologies presented are based on different physical principles and techniques for measurement and analysis, and in particular, some of them are compatible with the implementation in surgical forceps.

In particular, different body tissues are known to have different bioimpedance. The principle of so-called *dual energy X-ray absorptiometry* (DEXA) is to assess the water composition of a patient's body, to distinguish between lean mass and fat mass. Healthy tissue and cancer tissue have different bioimpedance values, and in [1] the possibility of evaluating the response to anti-cancer treatment based on the variation in bioimpedance is emphasized.

In contrast, a systematic review on electrical impedance tomography, i.e., an imaging technique that estimates the distribution of electrical conductivity in a body by measuring actual impedance values using surface electrodes, is presented in [2]. A device that can diagnose breast cancer by measuring bioimpedance, on the other hand, is described in [3].

Laser spectroscopy, thanks to the Raman effect, can also calculate a given typical value for a given tissue. In the recent review article [4], the role of laser spectroscopy in determining neoplastic tissue is described in detail. In a further article (see [5]), the problem is addressed more from a clinical point of view. In addition, through the estimation of optical properties (absorption and scattering), it is possible to trace the composition of the tissue, some related data about its microscopic structure, and functional information about the cell type. In [6] and [7], for example, patent papers are presented describing devices that can identify the presence of cancer in real time, based on laser spectroscopy and optical property analysis.

Measuring the concentration of certain gases, which are present more in one tissue than another, could be additional data to increase the accuracy of final identification and detection. Nitric oxide diffuses into the interstitial space of cancer cells, so the detection of this gas, performed by needle aspiration [8] and subsequent analysis, is an important additional data.

A further peculiarity of the neoplastic cell is the increase in membrane potential, which is managed by the ATP-ase pumps, i.e., enzymes capable of removing Na+ ions from the cell to favor the entry of K+ ions. The value of the resting potential varies, but in excitable cells is between -90 and -70 MV. The membrane potential in old cells goes down to -50MV and, in degenerate cells it goes down to - 15MV.

There are also known studies (see [9]) showing that the membrane potential tends to be more depolarized in cancer cells. A review on membrane biosensors can be found in the article [10], while the patent US9,534,999 (see also [11]) presents a technique that, using such biosensors, assesses the change in membrane potential and the associated heat release to map tumor tissue.

In recent years, a number of devices for capturing and projecting infrared images have also been protected as patent applications in order to more accurately locate cancerous tissue characterized by specific frequencies. The concept of image analysis of the lesion has also been proposed in patent US9,723,976 (see [12]), although in this case the presence of multiple cameras is provided within the device to compositely visualize the lesion itself to highlight peculiar pathological features.

However, imaging does not prove decisive in discriminating a cancerous tissue that has not been identified a priori.

It also seems appropriate to mention the U.S. Patent Application No. 16/057,720 (see [13]), which describes an algorithm that integrates data from multiple sensors without considering the metabolism of cancer and pathological cells.

Finally, reference is made to the Patent Application No. 16/729,790(see [15]), which integrates data from several sensors to assess "a state" (generic) of tissue. The invention refers to generic surgical systems, without expressing the purpose, either in the manner or in the characteristics of the systems themselves. Here, only the possibility of integrating an imaging system and a control circuit coupled to the imaging system, and a sensor capable of emitting and receiving electromagnetic radiation to be reprocessed based on the characteristics of the tissue under examination is described. Although this is a control circuit, the information received is always limited to a single physical feature of the suspected lesion, and may therefore be insufficient to allow automatic identification of the cancerous tissue.

Finally, the applicant has filed Italian patent number 102015000045150 on a sensorized surgical device that allows detection of the type of tissue examined, but does not allow the detection of possible cancerous tissue.

It seems apparent that the solutions indicated are onerous in terms of cost and operation time.

### Purpose of the invention

In light of the above, it is, therefore, the purpose of the present invention to propose a sensorized surgical device that can not only recognize tissue, but also determine the possible presence of cancerous tissue.

Another purpose of the invention is to propose a low cost device that can be easily integrated into known devices.

### Object of the invention

It is therefore specific object of the present invention a sensorized device for detecting conductive properties of an anatomical tissue, comprising a first jaw, a second jaw, mechanically connected to said first jaw and articulated with respect to it, so that said tissue can be caught between said two jaws, at least two application electrodes for applying an alternating current to said anatomical tissue, wherein said application electrodes are arranged on said jaws, at least two measurement electrodes, for detecting the impedance of said anatomical tissue, arranged on one of said jaws, a light source for emitting one or more light radiations on the anatomical tissue, and an optical receiver, configured to receive said light radiation to receive an optical spectrum of said anatomical tissue and generate a signal containing information on the optical spectrum, processing and control means, connected to said application electrodes and to said measurement electrodes, and signaling means, connected to said processing and control means, characterized in that said processing and control means are configured for: measuring the impedance between said measurement electrodes, when an alternating electric current is applied to the anatomical tissue by means of said application electrodes; and receiving said signal containing optical spectrum information; and in that said processing and control means are configured to process the signal from said measurement electrodes and said signal containing information on the optical spectrum using a neural network algorithm residing on a processor that has been trained to: extracting a characteristic value or pattern of impedance, determining diagnostic information relating to the anatomical tissue arranged between said jaws on the basis of said extracted characteristic impedance value or pattern and said detected optical spectrum; and wherein said processing and control means activate said signaling means to transmit a signal regarding the presence of cancerous tissue.

Always according to the invention, said processing and control means may be configured to measure impedance by detecting both the real part, associated with the resistance, and the imaginary part, associated with the capacity of said anatomical tissue.

Still according to the invention, said sensor may comprise a biosensor, connected to said processing and control means, and said processing and control means may be configured for measuring the change in membrane potential, extract a characteristic value or pattern of said membrane potential, and determining diagnostic information relating to the anatomical tissue arranged between said jaws based on said characteristic value or pattern of said sensed membrane potential, to determine the presence of cancer cells.

Advantageously according to the invention, said biosensor may consists of a pair of electrodes, each to be positioned in contact with the anatomical tissue to be examined.

Further according to the invention, said sensor may comprise further electrodes arranged on said jaws, connected to said processing and control means, and said processing and control means may be configured to measure the ratio between the module of the voltage between said measurement electrodes and the module of the alternating electric current applied to the anatomical tissue, and the difference between the phase of the voltage between said further electrodes and the phase of the alternating electric current applied to said anatomical tissue.

Always according to the invention, said neural network algorithm may be based on a neural network of the Convolutional Neural Network (CNN) type.

Further according to the invention, said processing and control means may comprise: an analysis unit connected to said measurement electrodes and to said application electrodes; and a control logic unit connected to said analysis unit, wherein said control logic unit processes the impedance between said measurement electrodes, when an alternating electric current is applied to the anatomical tissue by means of said application electrodes, and processes said signal containing information on the optical spectrum.

Preferably according to the invention, said sensor may comprise first focusing means for focusing the light radiation emitted by said light source, wherein said first focusing means are connected to said light source and are arranged on said first jaw, and second focusing means for focusing the light radiation received by said optical receiver, wherein said second focusing means are connected to said optical receiver and are arranged on said first jaw.

Additionally according to the invention, said diagnostic information are based on the fact that cancerous tissues, according to the type of pathology, have different resistance and/or capacity (impedance) values in the case of healthy or cancerous tissue.

Advantageously according to the invention, said analysis unit may also comprise a plurality of LEDs, each of which is associated with a specific signal to signal the possible presence of potential cancerous tissue.

Preferably according to the invention, said sensor may comprise a handle, and said analysis unit is arranged in said handle.

It is further object of the present invention a method for detecting conductive properties of an anatomical tissue by means of the sensorized device defined above, comprising the following steps: A. placing a tissue between said two jaws; B. applying an alternating current to said tissue by said application electrodes; C. detecting the impedance of said tissue by means of said at least two measurement electrodes; D. emitting one or more light radiations on the anatomical tissue by means of said light source; E. receiving an optical spectrum of said anatomical tissue and generating a signal containing information on the optical spectrum by means of said optical receiver; F. measuring the impedance between said measurement electrodes by means of said processing and control means; G. receiving said signal containing information on the optical spectrum by means of said processing and control means; H. processing by means of said processing and control means the signal from said analysis electrodes and said signal containing information on the optical spectrum using a neural network algorithm resident on a processor trained to: H1. extracting a characteristic impedance value or pattern; H2. determining diagnostic information relating to the anatomical tissue arranged between said jaws on the basis of said extracted characteristic impedance value or pattern and said detected optical spectrum; and I. activating said signaling means by means of processing and control means, so as to transmit a signal regarding the presence of cancerous tissue.

Always according to the invention, in said step H2 said processing and control means may discriminate portions of cancerous and non-cancerous tissue on the basis of the different energy state on the basis of the Warburg effect.

Still according to the invention, said step F further comprises the sub-step of measuring the variation of the membrane potential by means of said biosensor, and said step H further comprises the following sub-steps: H3. extracting a characteristic value or pattern of said membrane potential; and H4. determining diagnostic information relating to the tissue arranged between said jaws based on said characteristic value or pattern of said detected membrane potential, to determine the presence of cancer cells.

Advantageously according to the invention, in said step E, said signal containing information on the optical spectrum is based on the Raman effect.

It is also object of the present invention, a computer program comprising instructions which, when the program is executed by a computer, cause the computer to execute steps F-H of the method defined above.

It is also object of the present invention, a computer-readable storage medium comprising instructions which, when executed by a computer, cause the computer to execute steps F-H of the method defined above.

### Brief description of the figures

The present invention will be now described, for illustrative but not limitative purposes, according to its preferred embodiments, with particular reference to the figures of the enclosed drawings, wherein:
figure 1 shows a schematic view of a sensorized surgical device according to the present invention; and
figure 2 shows an operation diagram of the sensorized surgical device according to figure 1.

### Detailed description

In the various figures, similar parts will be indicated by the same numerical references.

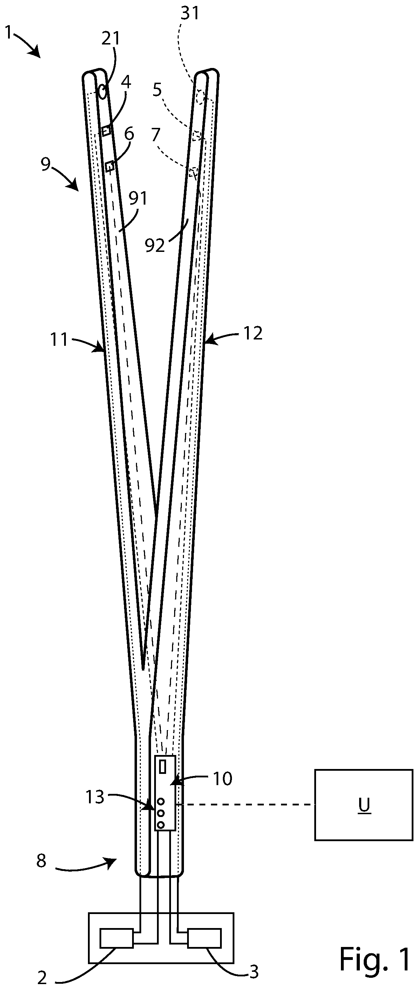

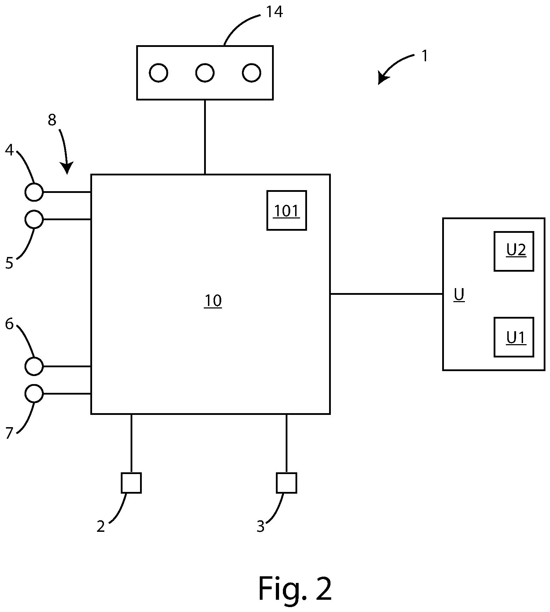

Referring to figures 1 and 2, a sensorized surgical device according to the present invention is observed, wholly indicated by numerical reference 1.

Sensorized surgical device 1 and the proposed methodology allow real-time diagnosis of the presence or absence of cancerous tissue on the organ under examination during surgery, providing an immediate response and providing the ability to perform the measurement on multiple contiguous specimens without the need for multiple biopsies, thereby significantly lowering the costs of using trained personnel and dedicated space.

The proposed solution extends the capabilities of other electro-optical devices for real-time identification of biological tissues, allowing the surgeon to verify in real time the type (cancerous or noncancerous) of the tissue being analyzed. The sensorized surgical device 1 involves, as better described below, several sensors or electrodes for real-time data collection, and an algorithm, implemented on hardware interfaced to the instrument itself, that allows fast and high probability identification of the possible cancerous nature of the tissue under analysis.

The sensorized surgical device 1 essentially comprises a first branch 11, a second branch 12, an analysis unit 10, to control the signals of the device 1, and a central control unit U to recognize an anatomical tissue by optical and electrical properties of the same and the possible presence of a cancerous tissue, based on impedance-metric properties.

The central control unit U is connected by wire or preferably wirelessly with the analysis unit 10. The central control unit U will be better described in the following, both structurally and functionally.

The sensorized surgical device 1 also comprises a light source 2 for emitting one or more light radiations on the anatomical tissue, connected to said analysis unit 10, and an optical receiver 3, connected to said analysis unit 10, configured to receive a light radiation, derive from said light radiation an optical spectrum of said anatomical tissue, and transmit to said analysis unit 10 a signal containing information about the optical spectrum. The analysis unit 10 is configured to transmit the signals to the central control unit U for their processing, as further explained below.

The sensorized surgical device 1 also comprises a pair of application electrodes 4 and 5. Each application electrode 4 and 5 has a free portion, to be placed in contact with the anatomical tissue to be examined. In addition, each of the two electrodes 4 and 5 is connected to the analysis unit 10 with cables 8. The analysis unit 10, by means of the application electrodes 4 and 5, is able to apply an alternating electric current to the anatomical tissue.

The sensorized surgical device 1 also comprises a pair of measurement electrodes 6 and 7, each having a portion to be placed in contact with the anatomical tissue to be examined. The two measurement electrodes 6 and 7 are also connected to the analysis unit 10, while the central control unit U can measure the impedance between said measurement electrodes 6 and 7 in terms of modulus and phase when an alternating electric current is applied to said anatomical tissue.

Light source 2 is activated by said analysis unit 10 so that the anatomical tissue is affected by one or more light radiations emitted by the light source. Each light radiation can have a respective predetermined wavelength.

The optical receiver 3 is electrically connected to said analysis unit 10 and is configured to receive at least the light radiation scattered by said anatomical tissue, having one or more wavelengths different from the predetermined wavelength of the light radiation emitted by the light source 2, as well as, as mentioned above, to extract the optical spectrum and send a signal containing the optical spectrum information to the analysis unit 10. The central control unit U is then configured to receive the signal sent by said optical receiver to said analysis unit 10, containing the optical spectrum information of the anatomical tissue.

Scattered light radiation is the portion of light radiation emitted by said light source 2 that has interacted, generally inelastically, with the molecular structure of the anatomical tissue.

Optical spectrum means an optical spectrum centered around the wavelength of light radiation emitted by light source 2.

In addition, light source 2 and optical receiver 3 are arranged externally to branches 11 and 12 of device 1.

In other embodiments, the sensorized surgical device 1 may not include the light source 2 and the optical receiver 3.

Light source 2 and optical receiver 3 can be placed inside surgical forceps 1, preferably inside the handle (not visible in the figure).

Light source 2 comprises first focusing means 21 to focus the light radiation emitted by light source 2. The first focusing means 21 are connected to light source 2.

The optical receiver 3 comprises second focusing means 31 for focusing the light radiation received by said optical receiver 3. The second focusing means 31 are connected to said optical receiver 3.

Said first focusing means 21 and said second focusing means 31 are arranged on said first jaw 11 and said second jaw 12, respectively.

The first electrode 4 and the second electrode 5 are placed on the first jaw 11 and the second jaw 12, respectively. When the sensorized surgical device 1 is in use, the two application electrodes 4 and 5 are placed so that at least a portion of anatomical tissue is present between them and said measurement electrodes 6 and 7, so that the alternating electric current flows from one electrode to the other, passing through the portion of anatomical tissue.

With reference to measurement electrodes 6 and 7, the central control unit U measures the impedance between them when an alternating electric current is applied through said analysis unit 10 to the anatomical tissue. The third electrode 6 and the fourth measurement electrode 7 are placed on the first jaw 11 and the second jaw 12, respectively.

In addition, the second end of each of said additional electrodes is electrically connected to the analysis unit 10.

When the sensorized surgical device 1 is in use, the measurement electrodes 6 and 7 are positioned so that anatomical tissue is present between them, and the impedance between said two further electrodes is measurable by the central control unit U, when an alternating electric current flows between said application electrodes 4 and 5, through the portion of the anatomical tissue under examination.

In other embodiments the application electrodes 4, 5, and measurement electrodes 6 and 7 can be arranged on a respective jaw 11 or 12 or on same jaws.

The sensorized surgical device 1 also comprises a biosensor 9 consisting of a pair of electrodes 91 and 92, each one to be placed in contact with the anatomical tissue to be examined. The two electrodes 91 and 92 are also connected to the analysis unit 10, which can measure the change of the membrane potential if cancer cells are present.

In other embodiments, the sensorized surgical device 1 may not include the biosensor 9 consisting of the electrodes 91 and 92.

The analysis unit 10 also comprises a plurality of LEDs 13, each one having a distinct color and in each case associated with a specific signal, e.g., type of tissue to be detected, or presence of potential cancerous tissue.

Cancerous tissues, depending on the type of disease, have different values or patterns of resistance and/or capacitance (impedance) in the case of healthy or cancerous tissue. As an example, for esophageal cancer, the resistance value increases progressively with the development of the disease compared with the case of healthy tissue. In the case of thyroid nodules, on the other hand, capacitance has higher average values in the case of cancerous tissue, while resistance takes on comparable values.

Therefore, there are different dynamic impedance patterns according to the type of pathology to be diagnosed and evaluated.

By means of the two additional measurement electrodes 6 and 7, the central control unit U is able to know the voltage (in terms of modulus and phase) between the two application electrodes 4 and 5, when an alternating electric current is applied to said anatomical tissue by means of said application electrodes 4 and 5. Also, again by means of said application electrodes 6 and 7, the analysis unit is able to detect the impedance between them.

The recognition unit 10, by means of the central control unit U, is configured to apply an alternating electric current to said anatomical tissue by means of said application electrodes 4 and 5, measure a voltage between said measurement electrodes 6 and 7 (in terms of modulus and phase), when said alternating electric current is applied to said anatomical tissue by means of said application electrodes 4 and 5, to receive signals containing information about said alternating electric current applied to said anatomical tissue by means of said application electrodes 4 and 5, and signals containing information about the corresponding voltage between further electrodes 6 and 7, as well as to calculate an impedance value of said anatomical tissue given by the ratio of said voltage to said alternating electric current.

It is important to be able to evaluate impedance in modulus and phase, so that both the real part (associated with resistance) and the imaginary part (associated with capacitance) can be evaluated, since for different forms of cancer there can be variations for resistance or capacitance alone.

The central control unit U is configured to measure the relationship between the voltage modulus between said measuring electrodes and the modulus of the alternating electric current applied to the anatomical tissue, as well as the phase difference between the voltage phase between said further electrodes and the phase of the alternating electric current applied to the anatomical tissue.

In addition, the recognition unit 10 can vary the frequency value of said alternating electric current to identify the anatomical tissue with a high probability.

In some embodiments, the recognition unit 10 is configured to apply, under the control of central control unit U, an additional alternating electric current to the same anatomical tissue via the two application electrodes 4 and 5. This additional alternating electric current has a predetermined frequency, different from the above predetermined frequency.

The central control unit U is thus configured to measure an additional voltage between the additional measurement electrodes 6 and 7 in terms of modulus and phase, when the additional alternating electric current is applied to said anatomical tissue, and calculate an additional impedance value given by the ratio of said additional voltage measured through said additional electrodes 6 and 7, when said additional alternating current is applied to said anatomical tissue, to the additional alternating electric current.

Again, depending on the value of the frequency of the alternating electric current, it is possible to discriminate specifically the contribution given by the real part of the impedance, or the contribution given by the imaginary part of the impedance. For the sake of completeness, it is reminded that impedance, in fact, is a physical vector quantity expressible as a complex number having a real part and an imaginary part. The real part of the impedance is associated with the information about the resistance of the anatomical tissue, and the imaginary part is associated with the information about the reactance of the anatomical tissue.

With a frequency between 100Hz and 1 KHz, it is possible to discriminate both the imaginary part of the impedance and the real part of the impedance, obtaining information on the reactance and resistance of the anatomical tissue, respectively.

The central control unit U is also configured to receive signals sent by said optical receiver 3 containing information about the optical spectrum of the anatomical tissue to be recognized.

The received optical spectrum takes on different characteristics in the case of healthy and cancerous tissue. As an example, in the case of lung cancer, the Raman shift peak observed at 2850 cm⁻¹, associated with fatty acids and lipids, presents much lower intensity in the case of cancerous tissue. In contrast, for gastric cancer, the Raman peaks present significantly higher intensity at 875 cm⁻¹ and 1745 cm⁻¹ for healthy tissue than for cancerous tissue.

The signal sent by said optical receiver 3 to said recognition unit 10 is an electrical signal. The signals from said electrodes 4 and 5 to said recognition unit 10 and the signals from said further electrodes 6 and 7 to said recognition unit 10 are also electrical signals.

The recognition unit 10, under the control of central control unit U, is configured to pass a current pulse between electrode pair 91 and 92 of biosensor 9 and read the voltage at the ends.

Having obtained the optical spectrum, the impedance value (in terms of the real and imaginary part) of the anatomical tissue to be recognized, and the membrane potential, the central control unit U is configured to perform in the present embodiment up to four comparisons, one concerning said optical spectrum, one concerning the membrane potential, and the other 2 concerning the real and imaginary part of the impedance, in order to recognize said anatomical tissue with a high probability.

For this purpose, the central control unit U comprises a database U2 in which data, such as the reference names of a plurality of predetermined anatomical tissues, a plurality of reference optical spectra, each of which is associated with a respective predetermined anatomical tissue, the values of a plurality of reference impedances, each of which is associated with a respective predetermined anatomical tissue, are stored.

Thus, each predetermined anatomical tissue or type of neoplasm or cancer is stored in the U2 database and associated (according to the tissue) with a respective reference optical spectrum and reference impedance value.

It is considered that an optical reference spectrum means an optical spectrum centered around a respective wavelength of light radiation emitted by a reference light source.

Specifically, said central control unit U is configured to perform a first comparison between the optical spectrum of said anatomical tissue to be recognized, and one or more reference optical spectra stored in said database, and to perform a second comparison between the impedance value obtained by said central control unit U and one or more reference impedance values stored in said database, as well as to identify said anatomical tissue from the combination of said two comparisons as well as the possible presence of cancerous tissue.

The central control unit U is then capable of recognizing whether an anatomical tissue has cancerous cells from a twofold comparison with data stored in the database.

From the optical spectrum it is possible to obtain information on the molecular structure of the anatomical tissue, while from the impedance value it is possible to obtain information on the resistance and reactance of the anatomical tissue, connected with the molecular structure of the anatomical tissue itself, with respect to the predetermined frequency of the alternating electric current applied to the anatomical tissue.

The combination of the information on the molecular structure of the anatomical tissue, given by the optical spectrum, and the information on the resistance and reactance of the same anatomical tissue, given by the impedance, allows both the recognition of the anatomical tissue and the determination of the possible presence of cancerous tissue.

According to the invention, the recognition unit 10 can be configured to perform a third comparison between said additional impedance value, mentioned above, and one or more reference impedance values stored in the database U2, to eliminate possible ambiguities of identification of said anatomical tissue in case of two anatomical tissues have similar electrical properties.

The operation of the sensorized surgical device 1 described above is as follows.

When jaws 11 and 12 are applied to a tissue to be examined, an alternating electric current is activated, similar to the above, on said anatomical tissue by means of application electrodes 4 and 5.

This electric current has a predetermined frequency, in which the intensity default value is less than 1 mA so as not to damage the anatomical tissue. In addition, the alternating electric current has a predetermined frequency between 100Hz and 10MHz.

This application is made by means of the analysis unit 10. Then, by means of the central control unit U, a voltage is measured between the measurement electrodes 6 and 7 in terms of modulus and phase, when alternating electric current is applied to the anatomical tissue by means of the application electrodes 4 and 5.

In addition, a signal is sent via a light source 2 to the optical receiver 3 containing the optical spectrum information of said anatomical tissue.

The central control unit U calculates or determines an anatomical tissue impedance value given by the ratio between the voltage and alternate electric current.

In one embodiment, similar to what is described in the Italian patent 102015000045150, a comparison is made between the optical spectrum of said anatomical tissue and reference optical spectra, as well as between the detected or calculated impedance value of the anatomical tissue under consideration and said plurality of reference impedance values, for the purpose of identifying the anatomical tissue based on these comparisons.

As mentioned, in addition to the above, an impedance analysis is performed. In particular, it is known that different body tissues have different bioimpedance. In fact, the working principle of so-called dual energy X-ray absorptiometry (DEXA) is to assess the body's water composition, to distinguish between lean mass and fat mass.

The cancer tissue has a different response to bioimpedance, compared with healthy tissue. This analysis is conducted through the analysis electrodes 4 and 5 and measurement electrodes 6 and 7, obtaining useful information for the identification of cancer tissue in real time.

The analysis of optical signals as described above is done through the estimation of optical properties (absorption and scattering) and spectral analysis using the Raman effect, so that it is possible to trace the composition of the tissue and some information about its microscopic structure. The transmission of the optical signals and the subsequent reception and analysis of the signals received downstream of the interaction with the tissue therefore make it possible to collect data to be used for the identification, as mentioned above. In this way, the sensor device 1 recognizes the type of tissue.

In addition, the sensorized surgical device 1 performs an analysis of the membrane potential and heat release, which is a peculiarity of the neoplastic cell due to the increased membrane potential. The value of the resting potential varies and, in degenerated cells, tends to be more depolarized than in healthy cells. These data can be retrieved through measurements of electric current and voltage. This processing is carried out by the central control unit U. The additional information available, makes it possible to obtain additional indicators of the cancerousness of the tissue under analysis.

The interfacing between the sensorized surgical device 1 and the central control unit U can be, as mentioned, either through wired or, preferably, wireless connections. The central control unit U, in addition to including the processing algorithms, also controls and synchronizes the overall process through the analysis unit 10, which involves:
- the injection of electrical and optical signals into the tissue;
- the reception and high-speed conversion to digital;
- the signal and data processing;
- the reporting of the outcome of the analysis to the surgeon through LEDs 13, preferably of different colors.

The central control unit U is capable of digitalizing received signals with a sampling rate of at least 100 MS/s and a resolution of at least 8 bits, but at the same time has limited power consumption.

In the sensorized surgical device 1, the central control unit U capable of discriminating a cancerous tissue from a healthy tissue by improving the response time through hardware implementation, i.e., in the processor U1, of a recognition algorithm, which implements neural networks.

To arrive at the recognition of a cancerous tissue, the analysis unit 10 together with the central control unit U, enable effective discrimination of cancerous and non-cancerous portions of tissue. More in detail, the discriminatory ability is related to the Warburg effect, which is peculiar to cancerous tissue compared with healthy tissue. In other words, the different energy state of cancerous tissue, which unlike healthy tissue produces predominantly energy with a high percentage of glycolysis, which, however, is not followed by the usual cycle of mitochondrial respiration, albeit in the presence of adequate oxygen concentration, determines the specificity of bio-impedance characteristics, uptake, diffusion and especially membrane potential variation, which therefore tends to be more depolarized in cancerous tissue than in healthy cells.

The Warburg effect is based, in fact, on the so-called "aerobic glycolysis", i.e., an essential aspect of the cancer cell metabolism based on the tendency to rely on glycolysis for energy production to a greater extent than in healthy cells. The increased glycolysis of cancer cells allows only partial oxidation of glucose molecules and is therefore poorly energy efficient.

Regarding the size of the examination areas, it is referred to the ergonomics and standard dimensions of surgical forceps and devices, which, on average, can grasp firmly 1.5 mm wide tissue.

At the same time, to reduce the response time, in the present invention the data processing is carried out in the analysis unit by means of a previously trained neural network implemented in the central control unit U.

The neural network is preferably of the CNN type, *Convolutional Neural* Network, although in other embodiments different types of networks can be used, such as MLP, *Multilayer Perceptron* networks. The training of the neural network is carried out on a hardware platform, i.e., the external dedicated central control unit U, connected, as mentioned, by wire or wireless, based on clinical data collected on both healthy and diseased portions of tissue.

The data collected through commercial instrumentation on bioimpedance, optical absorption and scattering properties, membrane potential, and heat release enable identification of the structure and weights of neurons in the network optimal for recognition.

The trained neural network is implemented in the central control unit U, which is a hardware and enables faster response times than a software-based solution. Therefore, the processing is carried out on a dedicated board U2 that houses a state-of-the-art SoC containing both a microprocessor with an embedded Linux system and an FPGA. The microprocessor U1 of said central control unit U enables the generation of the required driving signals for the application electrodes 4, 5 and the collection of data produced on the tissue under test; the collected data are then sent to the FPGA of the board U2 for real-time hardware processing. Finally, the response (binary) signal allows the surgeon to be notified of the outcome of the analysis by turning on one of the LEDs 13, controlled by the analysis unit 10, e.g., a red LED (in case of cancerous tissue) or a green LED (in case of healthy tissue).

### Advantages

An advantage of the present invention is the integration and the possibility of metabolism of tumor and pathological cells to increase the probability of correctly identifying cancerous tissue. Indeed, the advantage of the solution according to the invention consists of the processing that can provide, as much as possible, an operator-independent result, with objective data derived from impedance analysis, from the analysis of optical signals and not of images interpretable by the more or less experienced eye of a physician, from the analysis of membrane potential and heat release, which is, the latter, a peculiar aspect of the neoplastic cell, endowed with increased membrane potential (see also [14]). The integration of bioimpedance and biochemical data are thus a significant advantage for an evaluation of the cancer cell in toto that our inventive proposal brings to increase the objectivity of measurement.

An additional advantage of the present invention is to obtain real-time diagnosis of the possible presence of cancer cells in an anatomical tissue, avoiding the time-consuming clinical analysis.

### Referenze

[1] Brantlov S, J0dal L, Heitmann BL, Ward LC. Clinica! value of bioimpedance during long-term cancer therapy. Clin Case Rep J. 2021;2(5):l-6.
[2] Gomez-Cortés JC, Diaz-Carmona JJ, Padilla-Medina JA, Calderon AE, Gutiérrez AIB, Gutiérrez L6pez M, Prado-Olivarez J. Electrical Impedance Tomography Technical Contributions for Detection and 3D Geometry Localization of Breast Tumors: A Systematic Review. Micromachines (Basel). 2022 Mar 23;13(4):496. doi: 10.3390/mi13040496. PMID: 35457801; PMCID: PMC9025021.
[3] Davies, R. J. "Electrical bioimpedance analysis as a biomarker of breast density and/or breast cancer risk." U.S. PatentNo. 8,738,124.May 27, 2014.
[4] Khan MN, Wang Q, Idrees BS, Xiangli W, Teng G, Cui X, Zhao Z, Wei K and Abrar M (2022) A Review on Laser-Induced Breakdown Spectroscopy in Different _Cancers Diagnosis and Classification. Front. Phys. 10:821057. doi: 10.3389/fphy.2022.821057.
[5] Santos IP, Barroso EM, Bakker Schut TC, Caspers PJ, van Lanschot CGF, Choi DH, van der Kamp MF, Smits RWH, van Doom R, Verdijk RM, Noordhoek Hegt V, von der Thiisen JH, van Deurzen CHM, Koppert LB, van Leenders GJLH, Ewing-Graham PC, van Doom HC, Dirven CMF, Busstra MB, Hardillo J, Sewnaik A, Ten Hove I, Mast H, Monserez DA, Meeuwis C, Nijsten T, Wolvius EB, Baatenburg de Jong RJ, Puppels GJ, Koljenovié S. Raman spectroscopy for cancer detèction and cancer surgery guidance: translation to the clinics. Analyst. 2017 Aug 21;142(17):3025-3047. doi: 10.1039/c7an00957g. PMID: 28726868.
[6] Anderson, T. A., et al. "Optical sensor for needle-tip tissue identification and diagnosis." U.S. Patent Application No. 15/389,770.
[7] Huang, Z., et al. "Methods related to real-time cancer diagnostics at endoscopy utilizing fiber-optic Raman spectroscopy." U.S. Patent No. 9,931,039.3 Apr. 2018.
[8] Anbar, M. "Detection of cancerous lesions by measuring nitric mcide concentrations in tissue." U.S. Patent No. 6,035,225. Mar. 7, 2000.
[9] Berzingi, S., Newman, M., & Yu, H. G. (2016). Altering bioelectricity on inhibition of human breast cancer cells. Cancer cell intemational, 16(1), 1-9.
[10] Shen, Y. X., Saboe, P. O., Sines, I. T., Erbakan, M., & Kumar, M. (2014). Biomimetic membranes: A review. Joumal of Membrane Science, 454, 359-381.https://doi.org/10.1016/j.memsci.2013.12.019.
[11] Chen, E. T. "Nanostructured Biomimetic device for detecting a cancer cell or cancer cells." U.S. Patent No. 9,534,999. 3 Jan. 2017.
[12] Tesar, J. Optics for video camera on a surgical visualization system. U.S. Patent No 9,723,976, 2017.
[13] Doyle, Timothy E., et al. Apparatus, system and method for diagnostic imaging forceps. U.S. Patent Application No. 16/057,720, 2019.
[14] Nemeth, D.V.; Baldini, E.; Sorrenti, S.; D'Andrea, V.; Bellini, M.I. Cancer Metabolism and Ischemia-Reperfusion Injury: Two Sides of the Same Coin. J Clin. Med. 2022, 11, 5096. https://doi.org/10.3390/jcml 1175096.
[15] Shelton IV, Frederick E.; Deck, Andrew C. Surgical system control based on multiple sensed parameters. U.S. Patent Application No 16/729,790, 2021.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Sensorized device (1) for detecting conductive properties of an anatomical tissue, comprising
a first jaw (11),
a second jaw (12), mechanically connected to said first jaw (11) and articulated with respect to it, so that said tissue can be caught between said two jaws (11, 12),
at least two application electrodes (4, 5) for applying an alternating current to said anatomical tissue, wherein said application electrodes (4, 5) are arranged on said jaws (11, 12),
at least two measurement electrodes (6, 7), for detecting the impedance of said anatomical tissue, arranged on one of said jaws (11, 12),
a light source (2) for emitting one or more light radiations on the anatomical tissue, and
an optical receiver (3), configured to receive said light radiation to receive an optical spectrum of said anatomical tissue and generate a signal containing information on the optical spectrum,
processing and control means (10, U), connected to said application electrodes (4, 5) and to said measurement electrodes (6, 7), and
signaling means (13), connected to said processing and control means (10, U),
**characterized**
**in that** said processing and control means (10, U) are configured for:
measuring the impedance between said measurement electrodes (6, 7), when an alternating electric current is applied to the anatomical tissue by means of said application electrodes (4, 5); and
receiving said signal containing optical spectrum information; and
**in that** said processing and control means (10, U) are configured to process the signal from said measurement electrodes (6, 7) and said signal containing information on the optical spectrum using a neural network algorithm residing on a processor (U1) that has been trained to:
extracting a characteristic value or pattern of impedance,
determining diagnostic information relating to the anatomical tissue arranged between said jaws (11, 12) on the basis of said extracted characteristic impedance value or pattern and said detected optical spectrum; and
wherein said processing and control means (10, U) activate said signaling means (13) to transmit a signal regarding the presence of cancerous tissue.

2. Sensorized device (1) according to claim 1, **characterized in that** said processing and control means (10, U) are configured to measure impedance by detecting both the real part, associated with the resistance, and the imaginary part, associated with the capacity of said anatomical tissue.

3. Sensor device (1) according to any one of the preceding claims, **characterized**
**in that** it comprises a biosensor (9), connected to said processing and control means (10, U), and
**in that** said processing and control means (10, U) are configured for
measuring the change in membrane potential,
extract a characteristic value or pattern of said membrane potential, and
determining diagnostic information relating to the anatomical tissue arranged between said jaws (11, 12) based on said characteristic value or pattern of said sensed membrane potential, to determine the presence of cancer cells.

4. Sensorized device (1) according to the preceding claim, **characterized in that** said biosensor (9) consists of a pair of electrodes (91, 92), each to be positioned in contact with the anatomical tissue to be examined.

5. Sensor device (1) according to any one of the preceding claims, **characterized**
**in that** it comprises further electrodes arranged on said jaws (11, 12), connected to said processing and control means (10, U), and
**in that** said processing and control means (10, U) are configured to measure the ratio between the module of the voltage between said measurement electrodes (6 and 7) and the module of the alternating electric current applied to the anatomical tissue, and the difference between the phase of the voltage between said further electrodes and the phase of the alternating electric current applied to said anatomical tissue.

6. Sensor device (1) according to any one of the preceding claims, **characterized in that** said neural network algorithm is based on a neural network of the Convolutional Neural Network (CNN) type.

7. Sensor device (1) according to any one of the preceding claims, **characterized in that** said processing and control means (10, U) comprise:
an analysis unit (10) connected to said measurement electrodes (6, 7) and to said application electrodes (4, 5); and
a control logic unit (U) connected to said analysis unit (10), wherein said control logic unit (U) processes the impedance between said measurement electrodes (6, 7), when an alternating electric current is applied to the anatomical tissue by means of said application electrodes (4, 5), and processes said signal containing information on the optical spectrum.

8. Sensor device (1) according to the preceding claim, **characterized in that** it comprises
first focusing means (21) for focusing the light radiation emitted by said light source (2), wherein said first focusing means (21) are connected to said light source (2) and are arranged on said first jaw (11), and
second focusing means (31) for focusing the light radiation received by said optical receiver (3), wherein said second focusing means (31) are connected to said optical receiver (3) and are arranged on said first jaw (11).

9. Sensorized device (1) according to any one of the preceding claims, **characterized in that** said diagnostic information are based on the fact that cancerous tissues, according to the type of pathology, have different resistance and/or capacity (impedance) values in the case of healthy or cancerous tissue.

10. Sensor device (1) according to any one of the preceding claims, **characterized in that** said analysis unit (10) also comprises a plurality of LEDs (13), each of which is associated with a specific signal to signal the possible presence of potential cancerous tissue.

11. Sensor device (1) according to any one of the preceding claims, **characterized**
**in that** it comprises a handle, and
**in that** said analysis unit (10) is arranged in said handle.

12. Method for detecting conductive properties of an anatomical tissue by means of the sensorized device (1) according to any one of claims 1-11, comprising the following steps:
A. placing a tissue between said two jaws (11, 12);
B. applying an alternating current to said tissue by said application electrodes (4, 5);
C. detecting the impedance of said tissue by means of said at least two measurement electrodes (6, 7);
D. emitting one or more light radiations on the anatomical tissue by means of said light source (2);
E. receiving an optical spectrum of said anatomical tissue and generating a signal containing information on the optical spectrum by means of said optical receiver (3);
F. measuring the impedance between said measurement electrodes (6, 7) by means of said processing and control means (10, U);
G. receiving said signal containing information on the optical spectrum by means of said processing and control means (10, U);
H. processing by means of said processing and control means (10, U) the signal from said analysis electrodes (6, 7) and said signal containing information on the optical spectrum using a neural network algorithm resident on a processor (U1) trained to:
H1. extracting a characteristic impedance value or pattern;
H2. determining diagnostic information relating to the anatomical tissue arranged between said jaws (11, 12) on the basis of said extracted characteristic impedance value or pattern and said detected optical spectrum; and
I. activating said signaling means (13) by means of processing and control means (10, U), so as to transmit a signal regarding the presence of cancerous tissue.

13. Method according to the preceding claim, **characterized in that** in said phase H2 said processing and control means (10, U) discriminate portions of cancerous and non-cancerous tissue on the basis of the different energy state on the basis of the Warburg effect.

14. Method according to any one of claims 12 or 13, **characterized**
**in that** said step F further comprises the sub-step of measuring the variation of the membrane potential by means of said biosensor (9), and
**in that** said phase H further comprises the following sub-phases:
H3. extracting a characteristic value or pattern of said membrane potential; and
H4. determining diagnostic information relating to the tissue arranged between said jaws (11, 12) based on said characteristic value or pattern of said detected membrane potential, to determine the presence of cancer cells.

15. Method according to any one of claims 12 - 14, **characterized in that** in said step E, said signal containing information on the optical spectrum is based on the Raman effect.

16. A computer program comprising instructions which, when the program is executed by a computer, cause the computer to execute steps F-H of the method according to any one of claims 12-15.

17. A computer-readable storage medium comprising instructions which, when executed by a computer, cause the computer to execute steps F-H of the method according to any one of claims 12-15.
